(19) Europäisches Patentamt

European Patent Office

Office européen des brevets

(11) **EP 2 011 784 B1**

(12) # EUROPEAN PATENT SPECIFICATION

(45) Date of publication and mention of the grant of the patent:
**06.04.2016 Bulletin 2016/14**

(51) Int Cl.:
***C07C 319/12*** *(2006.01)*    ***C07C 323/52*** *(2006.01)*
***C08G 18/38*** *(2006.01)*    ***C08G 18/76*** *(2006.01)*

(21) Application number: **07737057.5**

(22) Date of filing: **12.04.2007**

(86) International application number:
**PCT/JP2007/000400**

(87) International publication number:
**WO 2007/122810 (01.11.2007 Gazette 2007/44)**

(54) **PROCESS FOR PRODUCING A PENTAERYTHRITOL ESTER OF 3-MERCAPTOCARBOXYLIC ACID**

VERFAHREN ZUR HERSTELLUNG EINES PENTAERYTHRITOL-ESTERS DER 3-MERCAPTOPROPIONSÄURE

PROCEDE DE PRODUCTION D'UN ESTER DE PENTAERYTHRITOL DE L'ACIDE 3-MERCAPTOPROPIONIQUE

(84) Designated Contracting States:
**DE FR GB IT**

(30) Priority: **21.04.2006 JP 2006117641**

(43) Date of publication of application:
**07.01.2009 Bulletin 2009/02**

(60) Divisional application:
**15156037.2 / 2 899 181**

(73) Proprietor: **Mitsui Chemicals, Inc.**
**Tokyo 105-7117 (JP)**

(72) Inventors:
• **SAKATA, Michiharu**
**Omuta-shi**
**Fukuoka 836-8610 (JP)**
• **KUMA, Shigetoshi**
**Omuta-shi**
**Fukuoka 836-8610 (JP)**

• **KOBAYASHI, Seiichi**
**Omuta-shi**
**Fukuoka 836-8610 (JP)**

(74) Representative: **Wills, Andrew Jonathan**
**Mewburn Ellis LLP**
**City Tower**
**40 Basinghall Street**
**London EC2V 5DE (GB)**

(56) References cited:
**JP-A- 10 120 646       JP-A- 11 080 117**
**JP-A- 2001 039 945     JP-A- 2005 336 104**

Remarks:
The file contains technical information submitted after the application was filed and not included in this specification

**Description**

TECHNICAL FIELD

The present invention relates to a process for producing pentaerythritol mercaptocarboxylic esters.

BACKGROUND ART

Plastic lenses are light weight, less broken, and dyeable, as compared with inorganic lenses. Therefore, in recent years, the application of the plastic lenses to optical materials such as spectacle lens or a camera lens has increased rapidly.

The resins for the plastic lenses have been required to have new excellent performances such as a high refractive index, a high Abbe's number, low specific gravity, and high heat resistance. A variety of resin materials for lenses have been developed and used until now.

Among them, there have been actively proposed polythiourethane-based resins, and the present inventors have also proposed various plastic lenses obtained by using these polythiourethane-based resins (see Patent Documents 1, 2, and 3).

Among the polythiourethane-based resins, as the most typical resin, a polyurethane based resin obtained by polymerizing pentaerythritol mercaptocarboxylic ester with a polyiso(thio)cyanate compound is colorless and transparent, has a high refractive index and low dispersion, is excellent in impact resistance, dyeabiltiy, processability and the like, and is one of resins which are optimum for plastic lenses.

Pentaerythritol mercaptocarboxylic ester is produced by a so-called direct esterification method. For example, the ester is produced by reacting a usual polyhydric alcohol with a mercaptocarboxylic acid in the presence of an esterifying catalyst, while removing by-produced water out of the system (Refer to Patent Document 4).

Pentaerythritol, one of starting materials of the pentaerythritol mercaptocarboxylic ester, is usually produced by subjecting acetaldehyde and formaldehyde to condensation. The purity of the pentaerythritol obtained by the appropriate production process is about 90 wt %, and pentaerythritol contains various kinds of impurities. One of such impurities is bispentaerythritol that is a condensation of two molecules of formaldehyde of pentaerythritol. When this bispentaerythritol is contained in pentaerythritol in excess of a specific amount, it has been known that there might possibly be problems such that it is difficult to be released from a mold after completion of polymerization with a polyiso(thio)cyanate compound in some cases, and bubbles are generated inside the obtained lens (refer to Patent Documents 5 and 6).

With respect to such pentaerythritol, one of starting materials of pentaerythritol mercaptocarboxylic ester, there have been shown a correlation between its quality and impurities and the quality of the obtained lens in several documents. However, there has been scarcely known a correlation between the quality of the other starting material mercaptocarboxylic acid and the quality of the obtained lens.

As one mercaptocarboxylic acid, 3-mercaptopropionic acid can be cited. Since 3-mercaptopropionic acid has extremely bad storage stability, it has been known that the purity is easily lowered due to the contact with oxygen in the air or storage temperature so that the content of impurities is increased. Furthermore, the melting point of 3-mercaptopropionic acid is low (16.8 degree centigrade) so when the storage temperature becomes low, particularly in winter, the acid is solidified in some cases. Since a liquid is more easily handled than a solid, the 3-mercaptopropionic acid is kept liquid by heating, for the acid is handled by heat-melting in advance when it is solidified in many cases. However, when the acid is excessively heated for storage by melting or heating, it causes a decrease in the purity. When pentaerythritol 3-mercaptopropionic ester is produced by using such a 3-mercaptopropionic acid and is employed for a long period of time, the quality of the obtained pentaerythritol 3-mercaptopropionic ester is not regular and the color is deteriorated in some cases even if the production conditions are the same. The viscosity of the polymerizable composition before polymerization obtained by mixing the pentaerythritol 3-mercaptopropionic ester with a polyiso(thio)cyanate compound is high so that it becomes difficult to handle the composition such that a) in the degassing step of the lens process, bubbles are hardly removed, b) in the filtering step for removing foreign substances, it takes time and filtering cannot be performed, c) injection into a mold cannot be done and the like. Furthermore, a lens obtained by the polymerizable composition has problems of deterioration in the color, whitening and the like.

Accordingly, it has been demanded that deterioration in the color of pentaerythritol mercaptocarboxylic ester, an increase in the viscosity of the polymerizable composition before polymerization with a polyiso(thio)cyanate compound, and deterioration in the color or whitening of the lens should be suppressed.

Patent Document 7 discloses a process for the esterification of a mercaptocarboxylic acid with pentaerythritol, wherein the sodium, calcium and bispentaerythritol content of the pentaerythritol are limited.

Patent Document 1: Japanese Patent Laid-open No. S60(1985)-199016
Patent Document 2: Japanese Patent Laid-open No. S60(1985)-217229

Patent Document 3: Japanese Patent Laid-open No. S63(1988)-46213
Patent Document 4: Japanese Patent Publication No. S39(1964)-9071
Patent Document 5: Japanese Patent Laid-open No. S56(1981)-20530
Patent Document 6: Japanese Patent Laid-open No. H10(1998)-120646
Patent Document 7: Japanese Patent Laid-open No. 2005-336104

## DISCLOSURE OF THE INVENTION

An object of the present invention is to obtain colorless and transparent pentaerythritol mercaptocarboxylic ester when pentaerythritol is reacted with a mercaptocarboxylic acid. Furthermore, the present invention makes it possible to provide a polymerizable composition having a low viscosity containing the pentaerythritol mercaptocarboxylic ester and polyiso(thio)cyanate, and to provide a polyurethane based resin which is colorless and transparent without causing whitening thereof by polymerizing the polymerizable composition.

In order to solve the above objects, the present inventors have conducted an extensive study and as a result, have determined that the cause of whitening of a polyurethane based resin is in the pentaerythritol mercaptocarboxylic ester as monomer thereof. The inventors have further continued an extensive study and as a result, surprisingly found that, when the pentaerythritol mercaptocarboxylic ester is produced using the mercaptocarboxylic acid 3-mercaptopropionic acid having a content of thioester formed by condensation of two molecules of the mercaptocarboxylic acid of not more than a specific amount as a starting material, the above problems are solved, and a polyurethane based resin which is colorless and transparent and in which the whitening is suppressed is obtained. Thus, the present invention has been completed.

That is, the present invention relates to:

(1) a process for producing pentaerythritol mercaptocarboxylic ester, comprising:

(i) purifying a mercaptocarboxylic acid to reduce the content of thioester formed by condensation of two molecules of the acid to not more than 5% in terms of area percentage, as determined by high-performance liquid chromatography with detection at 230 nm, of the total area of the mercaptocarboxylic acid and thioester formed by intermolecular condensation of the acid being taken as 100%; and (ii) reacting pentaerythritol with the purified mercaptocarboxylic acid wherein said mercaptocarboxylic acid is a 3-mercaptopropionic acid.

(2) the process as set forth in (1) above, in which the content of bispentaerythritol in said pentaerythritol is not more than 7 wt %, based on the total weight of pentaerythritol.

According to the production process of the present invention, colorless and transparent pentaerythritol mercaptocarboxylic ester is obtained. Furthermore, the polymerizable composition before polymerization obtained by mixing the pentaerythritol mercaptocarboxylic ester with a polyiso(thio)cyanate compound comes to have a low viscosity, while the polyurethane based resin obtained by polymerizing the polymerizable composition becomes a colorless and transparent resin in which whitening is suppressed.

## BEST MODE FOR CARRYING OUT THE INVENTION

The present invention will be illustrated in detail below.

In the purified mercaptocarboxylic acid, used as a starting material of pentaerythritol mercaptocarboxylic ester in the present invention, the content of thioester formed by condensation of two molecules ) of the mercaptocarboxylic acid is not more than a specific amount. Namely, there is used a mercaptocarboxylic acid whose content of thioester formed by condensation of two molecules of the acid is not more than 5% (in terms of area percentage) as determined by high-performance liquid chromatography, of the total area of the mercaptocarboxylic acid and thioester formed by being intermolecular condensation of the acid being taken as 100%. The mercaptocarboxylic acid is 3-mercaptopropionic acid.

Herein, the thioester formed by intermolecular condensation of the mercaptocarboxylic acid is a compound obtained by condensation of a mercapto group and a carboxyl group of the mercaptocarboxylic acid between molecules by a thioester bond, and a compound bonded between two molecules, three or more molecules. The compound Thus, the obtained by condensation of a mercapto group and a carboxyl group between two molecules by a thioester bond is referred to as thioester formed by condensation of two molecules. Thus, the thioester formed by intermolecular condensation of 3-mercaptopropionic acid is a 3-(3-mercaptopropanoylthio)propionic acid.

when When the content of thioester formed by condensation of two molecules of the mercaptocarboxylic acid is not more than 5% (in terms of area percentage) as determined by high-performance liquid chromatography when the total area of the mercaptocarboxylic acid and thioester formed by intermolecular condensation of the acid is taken as 100%, the color of the pentaerythritol mercaptocarboxylic ester produced by using the mercaptocarboxylic acid becomes colorless and transparent. Furthermore, the polymerizable composition before polymerization obtained by mixing the pentaerythritol mercaptocarboxylic ester with polyiso(thio)cyanate has a low viscosity, while the obtained polyurethane based resin becomes a colorless and transparent polyurethane based resin in which the whitening is suppressed. From the viewpoint of suppression of whitening, the content of thioester formed by condensation of two molecules of the mercaptocarboxylic acid used in the present invention is preferably from not less than 0.01% to not more than 5%, more preferably from not less than 0.01% to not more than 3%, and further preferably from not less than 0.01% to not more than 1% in terms of area percentage as determined by the high-performance liquid chromatography.

The content of thioester formed by condensation of two molecules illustrated in the present invention is measured, for example, by the following method. A high-performance liquid chromatography system (LC-6A, SPD-10A, CTO-10A, products of Shimadzu Corporation) is connected with a column Mightysil RP-18 GP (a product of Kanto Chemical Co., Inc.) and an aqueous solution of 0.01M $KH_2PO_4$/acetonitrile (40/60) is used as an eluent, and the content of thioester formed by condensation of two molecules in the mercaptocarboxylic acid is analyzed at a column temperature of 40 degree centigrade at a flow rate of the eluent of 0.95 ml/min. at a wavelength of 230 nm. The content of thioester formed by condensation of two molecules is defined by area percentage as determined by the high-performance liquid chromatography in the case of the total area of the mercaptocarboxylic acid and thioester formed by intermolecular condensation of the acid is taken as 100%.

In the mercaptocarboxylic acid, the increased content of thioester formed by intermolecular condensation of the mercaptocarboxylic acid is caused by a method of storing the mercaptocarboxylic acid. The generation of thioester formed by intermolecular condensation of the mercaptocarboxylic acid is accelerated by the entrainment of iron in the mercaptocarboxylic acid, the contact of the mercaptocarboxylic acid with oxygen in the air, and when the storage temperature becomes high. Accordingly, the mercaptocarboxylic acid is preferably kept at a state that the temperature is controlled to be low in a vessel free from the contact with iron, in a nitrogen atmosphere. For example, the temperature suitable for the storage is in the range of not less than 10 to not more than 60 degree centigrade, more preferably in the range of not less than 15 to not more than 50 degree centigrade, and further preferably in the range of not less than 20 to not more than 40 degree centigrade.

Further, by means of purification, the content of thioester formed by condensation of two molecules in the mercaptocarboxylic acid is reduced. The purification method is not particularly limited, but, for example, purification by distillation can be cited.

In the other starting material, pentaerythritol, the content of bispentaerythritol of impurities and further the content of metals are preferably not more than a specific amount. For example, the content of bispentaerythritol in pentaerythritol is preferably in the range of not less than 0.01 to not more than 7 wt %, more preferably in the range of not less than 0.1 to not more than 5 wt %, and further preferably in the range of not less than 1 to not more than 5 wt %, based on the total weight of pentaerythritol.

Examples of the metal include alkali metals such as Li, Na, K, Rb and Cs, alkaline earth metals such as Mg, Ca, Sr and Ba, and other metals including Ti, V, Cr, Mn, Fe, Co, Ni, Cu and Zn. Specifically, it is preferable that the content of alkali metal and alkaline earth metal, particularly Na and Ca, is suppressed.

When the content of bispentaerythritol is within the aforementioned range and the total content of metals is less than 1 wt % based on the total weight of pentaerythritol, the release property from the mold after completion of the polymerization of the obtained pentaerythritol mercaptocarboxylic ester with polyiso(thio)cyanate becomes good so that the occurrence of bubbles in the obtained polyurethane based resin can be suppressed.

In order to react pentaerythritol with 3-mercaptopropionic acid, as the esterifying catalyst which is usually used, for example, acid catalysts having typical examples of mineral acids such as sulfuric acid, hydrochloric acid, phosphoric acid and alumina, and organic acids such as p-toluenesulfonic acid, benzenesulfonic acid, methanesulfonic acid, trichloroacetic acid and dibutyl tin oxide are preferably used.

3-mercaptopropionic acid The preferable proportion of pentaerythritol and the 3-mercaptopropionic acid is not particularly limited, but the molar ratio is, for example, in the range of not less than 3.5 to not more than 6.0 (acid/pentaerythritol), more preferably in the range of not less than 3.8 to not more than 5.0, and further preferably in the range of not less than 4.0 to not more than 4.5. When the proportion is within the above range, it is possible to produce pentaerythritol mercaptocarboxylic ester having high purity with good efficiency. The obtained pentaerythritol mercaptocarboxylic ester is colorless and transparent, and comes to have a low viscosity, and the polymerizable composition containing the pentaerythritol mercaptocarboxylic ester and a polyiso(thio)cyanate compound also comes to have a low viscosity. The resin obtained by curing the polymerizable composition is excellent in the color, and has excellent qualities such as optical properties and heat resistance.

Meanwhile, as the preferable condition for the reaction of pentaerythritol with 3-mercaptopropionic acid, for example, the temperature is in the range of not less than 80 to not more than 140 degree centigrade, and more preferably in the range of not less than 100 to not more than 125 degree centigrade. When the temperature is within the above range, the reaction of pentaerythritol with a mercaptocarboxylic acid is further accelerated. The obtained pentaerythritol mercaptocarboxylic ester is colorless and transparent, and comes to have a low viscosity, while the polymerizable composition containing the pentaerythritol mercaptocarboxylic ester and a polyiso(thio)cyanate compound also comes to have a low viscosity. The resin obtained by curing the polymerizable composition is excellent in the color, and has excellent qualities such as optical properties and heat resistance.

To produce pentaerythritol mercaptocarboxylic ester, an azeotropic agent is not necessarily used. However, there is generally used a method including continuously removing by-produced water out of the system under heating reflux using an azeotropic agent. Examples of the azeotropic agent which is usually used include benzene, toluene, xylene, nitrobenzene, chlorobenzene, dichlorobenzene, anisole, diphenyl ether, methylene chloride, chloroform, dichloroethane and the like. These may be used singly, or two or more kinds thereof may be used in combination, or may be used in mixture with other solvents.

The pentaerythritol mercaptocarboxylic ester obtained by the aforementioned process is not particularly limited as long as it is a compound obtained by condensation of pentaerythritol with 3-mercaptopropionic acid.

Furthermore, these ester compounds may be compounds obtained by fully esterifying a hydroxy group of pentaerythritol or compounds obtained by esterifying only a part of a hydroxy group. Further, these ester compounds may be used singly, or two or more kinds thereof may be used in combination when a polyurethane based resin is obtained by polymerizing the ester compound with a polyiso(thio)cyanate compound.

The polyiso(thio)cyanate compound is not particularly limited as long as it is a compound having at least two or more iso(thio)cyanate groups in a molecule. Examples thereof include aliphatic polyisocyanate compounds such as

hexamethylene diisocyanate, 2,2-dimethylpentane diisocyanate, 2,2,4-trimethylhexane diisocyanate, butene diisocyanate, 1,3-butadiene-1,4-diisocyanate, 2,4,4-trimethylhexamethylene diisocyanate, 1,6,11-undecane triisocyanate, 1,3,6-hexamethylene triisocyanate, 1,8-diisocyanato-4-isocyanatomethyloctane, bis(isocyanatoethyl)carbonate, bis(isocyanatoethyl)ether, lysine diisocyanatomethyl ester and lysine triisocyanate;

polyisocyanate compounds having an aromatic compound such as 1,2-diisocyanatobenzene, 1,3-diisocyanatobenzene, 1,4-diisocyanatobenzene, 2,4-diisocyanatotoluene, ethylphenylene diisocyanate, isopropylphenylene diisocyanate, dimethylphenylene diisocyanate, diethylphenylene diisocyanate, diisopropylphenylene diisocyanate, trimethylbenzene triisocyanate, benzene triisocyanate, biphenyl diisocyanate, toluidine diisocyanate, 4,4'-methylenebis(phenyl isocyanate), 4,4'-methylenebis(2-methylphenyl isocyanate), bibenzyl-4,4'-diisocyanate, bis(isocyanatophenyl)ethylene, bis(isocyanatoethyl)benzene, bis(isocyanatopropyl)benzene, $\alpha,\alpha,\alpha',\alpha'$-tetramethylxylylene diisocyanate, bis(isocyanatobutyl)benzene, bis(isocyanatomethyl)naphthalene, bis(isocyanatomethylphenyl)ether, bis(isocyanatoethyl)phthalate, and 2,6-di(isocyanatomethyl)furan;

sulfur-containing aliphatic polyisocyanate compounds such as bis(isocyanatomethyl)sulfide, bis(isocyanatoethyl)sulfide, bis(isocyanatopropyl)sulfide, bis(isocyanatohexyl)sulfide, bis(isocyanatomethyl)sulfone, bis(isocyanatomethyl)disulfide, bis(isocyanatoethyl)disulfide, bis(isocyanatopropyl)disulfide, bis(isocyanatomethylthio)methane, bis(isocyanatoethylthio)methane, bis(isocyanatomethylthio)ethane, bis(isocyanatoethylthio)ethane, 1,5-diisocyanato-2-isocyanatomethyl-3-thiapentane, 1,2,3-tris(isocyanatomethylthio)propane, 1,2,3-tris(isocyanatoethylthio)propane, 3,5-dithia-1,2,6,7-heptane tetraisocyanate, 2,6-diisocyanatomethyl-3,5-dithia-1,7-heptane diisocyanate, 2,5-diisocyanate methyl thiophene and 4-isocyanatoethylthio-2,6-dithia-1,8-octane diisocyanate;

aromatic sulfide based polyisocyanate compounds such as 2-isocyanatophenyl-4-isocyanatophenyl sulfide, bis(4-isocyanatophenyl)sulfide and bis(4-isocyanatomethylphenyl)s/ulfide;

aromatic disulfide based polyisocyanate compounds such as bis(4-isocyanatophenyl)disulfide, bis(2-methyl-5-isocyanatophenyl)disulfide, bis(3-methyl-5-isocyanatophenyl)disulfide, bis(3-methyl-6-isocyanatophenyl)disulfide, bis(4-methyl-5-isocyanatophenyl)disulfide and bis(4-methoxy-3-isocyanatophenyl)disulfide;

sulfur-containing alicyclic polyisocyanate compounds such as 2,5-diisocyanatotetrahydrothiophene, 2,5-diisocyanatomethyltetrahydrothiophene, 3,4-diisocyanatomethyltetrahydrothiophene, 2,5-diisocyanato-1,4-dithiane, 2,5-diisocyanatomethyl-1,4-dithiane, 4,5-diisocyanato-1,3-dithiolane, 4,5-bis(isocyanatomethyl)-1,3-dithiolane and 4,5-diisocyanatomethyl-2-methyl-1,3-dithiolane;

aliphatic polyisothiocyanate compounds such as 1,2-diisothiocyanatoethane and 1,6-diisothiocyanatohexane; alicyclic polyisothiocyanate compounds such as cyclohexane diisothiocyanate; aromatic polyisothiocyanate compounds such as 1,2-diisothiocyanatobenzene, 1,3-diisothiocyanatobenzene, 1,4-diisothiocyanatobenzene, 2,4-diisothiocyanatotoluene, 2,5-diisothiocyanato-m-xylene, 4,4'-methylenebis(phenyl isothiocyanate), 4,4'-methylenebis(2-methylphenyl isothiocyanate), 4,4'-methylenebis(3-methylphenyl isothiocyanate), 4,4'-diisothiocyanatobenzophenone, 4,4'-diisothiocyanato-3,3'-dimethylbenzophenone and bis(4-isothiocyanatophenyl)ether;

further, carbonyl polyisothiocyanate compounds such as 1,3-benzenedicarbonyl diisothiocyanate, 1,4-benzenedicarbonyl diisothiocyanate and (2,2-pyridine)-4,4-dicarbonyl diisothiocyanate; sulfur-containing aliphatic polyisothiocyanate compounds such as thiobis(3-isothiocyanatopropane), thiobis(2-isothiocyanatoethane) and dithiobis (2-isothiocyanatoethane);

sulfur-containing aromatic polyisothiocyanate compounds such as 1-isothiocyanato-4-[(2-isothiocyanato)sulfonyl] benzene, thiobis(4-isothiocyanatobenzene), sulfonyl (4-isothiocyanatobenzene) and dithiobis (4-isothiocyanatobenzene); sulfur-containing alicyclic polyisothiocyanate compounds such as 2,5-diisothiocyanatothiophene and 2,5-diisothiocyanato-1,4-dithiane; and

compounds having an isocyanato group and an isothiocyanato group such as 1-isocyanato-6-isothiocyanatohexane, 1-isocyanato-4-isothiocyanatocyclohexane, 1-isocyanato-4-isothiocyanatobenzene, 4-methyl-3-isocyanato-1-isothiocyanatobenzene, 2-isocyanato-4,6-diisothiocyanato-1,3,5-triazine, 4-isocyanatophenyl-4-isothiocyanatophenyl sulfide and 2-isocyanatoethyl-2-isothiocyanatoethyl disulfide.

Furthermore, there can be used their halogen substituted compounds such as chlorine substituted compounds and bromine substituted compounds; their alkyl substituted compounds, their alkoxy substituted compounds, their nitro substituted compounds, prepolymer type modified compounds modified with polyhydric alcohols, carbodiimide-modified compounds, urea-modified compounds, biuret-modified compounds, or compounds obtained by dimerization or trimerization reactionss. These compounds may be used singly, or two or more compounds may be used in combination.

The proportion of the pentaerythritol mercaptocarboxylic ester and the polyiso (thio) cyanate compound is not particularly limited, but the molar ratio is usually in the range of not less than 0.3 to not more than 2.0 (SH group/NCO group), preferably in the range of not less than 0.7 to not more than 2.0, and further preferably in the range of not less than 0.8 to not more than 1.3. When the molar ratio is within the above range, the resin obtained by curing the polymerizable composition containing the pentaerythritol mercaptocarboxylic ester and the polyiso(thio)cyanate compound is excellent in color, and has excellent qualities such as optical properties and heat resistance.

For purposes of improvement of general properties, operability and polymerization reactivity of the polyurethane based resin, other substances may be added, in addition to the ester compound and iso(thio)cyanate compound forming the urethane resin. For example, in addition to a starting material for forming a urethane, one or two or more active hydrogen compounds such as amines, epoxy compounds, olefin compounds, carbonate compounds, ester compounds, metals, metal oxides, organic metal compounds and inorganic substances may be added.

Further, a variety of substances such as a chain extender, a crosslinking agent, a photostabilizer, a UV absorber, an antioxidant, an oil soluble dye, a filler, a releasing agent, and a blueing agent, may be added, depending on the purposes, as in a known molding method. In order to adjust to a desired reaction rate, a thiocarbamic acid S-alkyl ester or a known reaction catalyst used for producing polyurethane may be added as appropriate. The lens formed of the polyurethane resin can be usually obtained by casting polymerization.

Specifically, pentaerythritol mercaptocarboxylic ester is mixed with a polyiso(thio)cyanate compound. This mixed solution is degassed according to a proper method as needed, and then injected into a mold and usually slowly heated from a low temperature to a high temperature for polymerization.

The thus-obtained polyurethane based resin has a high refractive index, a low dispersion, excellent heat resistance and durability, light weight, and excellent impact resistance and the occurrence of whitening is further suppressed. Thereby it being suitable as an optical material and a transparent material, e.g. for a spectacle lens or a camera lens.

Furthermore, the lens which is obtained by using the polyurethane resin may be, if necessary, subjected to physical or chemical treatment such as surface abrasion treatment, antistatic treatment, hard coat treatment, non-reflective coat treatment, dyeing treatment and polarizing treatment, for prevention of reflection, enhancement of hardness, improvement of abrasion resistance, improvement of chemical resistance, supply of anticlouding or supply of fashionability.

EXAMPLES

The present invention is now illustrated in detail below with reference to Examples. In the following Examples and Comparative Examples 3-mercaptopropionic acid was used. The 3-mercaptopropionic acid was analyzed by the following method. The color of the obtained pentaerythritol 3-mercaptopropionic ester, the viscosity of a polymerizable composition before polymerization composed of pentaerythritol 3-mercaptopropionic ester and a polyiso(thio)cyanate compound, and the color and transparency of a polyurethane based resin obtained by polymerization were evaluated in the following test method.

· Content of 3-(3-mercaptopropanoylthio)propionic acid: A high-performance liquid chromatography system (LC-6A, SPD-10A, CTO-10A, products of Shimadzu Corporation) was connected with a column Mightysil RP-18GP (a product of Kanto Chemical Co., Inc.) and the content thereof was measured by the high-performance liquid chromatography. Specifically, using an aqueous solution of 0.01M $KH_2PO_4$/acetonitrile (40/60) as an eluent and dissolving the 3-mercaptopropionic acid in the eluent, area percentage of the 3-(3-mercaptopropanoylthio)propionic acid was analyzed at a column temperature of 40 degree centigrade at a flow rate of the eluent of 0.95 ml/min. at a wavelength of 230 nm in the case of the total area of the mercaptocarboxylic acid and thioester formed by intermolecular condensation of the acid was taken as 100%.

· Content of bispentaerythritol: Pentaerythritol was dissolved in water, and then the resulting aqueous solution was applied to the high-performance liquid chromatography to measure the content of bispentaerythritol.

· Content of sodium and calcium: Pentaerythritol was dissolved in water, and then the resulting aqueous solution was applied to the high-performance liquid ion chromatography to measure the content of sodium and calcium.

· Y.I. (yellow index) of pentaerythritol mercaptocarboxylic ester: Y.I. was employed as an analyzing item for evaluating the color of the pentaerythritol mercaptocarboxylic ester. The smaller the Y.I. value was, the better the color of the pentaerythritol mercaptocarboxylic ester was, while the greater the Y.I. value was, the worse the color was. Such a correlation was obtained. Using a colorimeter CT-210 (a product of Minolta Camera Co., Ltd.), tristimulus value Y and color coordination x, y on the CIE-1391 chromaticity diagram were measured. Firstly, distilled water was fed into a cell CT-A20 having an optical path length of 20 mm, and a white calibration was performed as Y=100.00, x=0.3101 and y=0.3162. Thereafter, a sample was fed into the same cell and the color measurement was carried out. The measurement results, x and y values, were used to calculate Y.I. according to the following formula:

$$Y.I. = (234 \times x + 106 \times y + 106)/y \qquad (1)$$

· Y.I. (yellow index) of polyurethane based resin: Y.I. was employed as an analyzing item for evaluating the color of a plastic lens containing a polyurethane based resin. The smaller the Y.I. value was, the better the color of the plastic lens was, while the greater the Y.I. value was, the worse the color was. Such a correlation was obtained. The plastic lens of a circular flat plate having a thickness of 9 mm and $\varphi$75 mm was prepared, and chromaticity coordinates x and y were measured by using a colorimeter CT-210 (a product of Minolta Camera Co., Ltd. ). The measurement results, x and y values, were used to calculate Y.I. according to the above formula (1).

· Loss degree of transparency: As an analyzing item for evaluating the transparency of the plastic lens containing a polyurethane based resin, the loss degree of transparency was employed. The loss degree of transparency was obtained in the following means. The lens plate of a circular flat plate having a thickness of 9 mm and $\varphi$75 mm was prepared. Then, the lens plate was irradiated with a light source (Luminar Ace LA-150A, a product of Hayashi Watch Works Co., Ltd.) for measuring the loss degree of transparency with a gray scale image processing unit. Captured images were expressed in numbers by gray scale image processing to obtain the loss degree of transparency. When the loss degree of transparency is not more than 50, it was indicated with o, while, when it was greater than 50, it was indicated with x.

Example 1

Synthesis of pentaerythritol 3-mercaptopropionic ester

To a 2-liter, 4-necked flask equipped with a stirrer, a reflux condensing water separator, a nitrogen gas purge tube and a thermometer were added 663.0 parts by weight (6.23 mol) of a 3-mercaptopropionic acid with the purity of 99.7% containing 0.2% (in terms of area percentage) of 3-(3-mercaptopropanoylthio)propionic acid, 204.6 parts by weight (1.5 mol) of pentaerythritol with the purity of 95.2% containing 4.7 wt % of bispentaerythritol, 0.1 wt % of sodium and 0.02 wt % of calcium, 5.7 parts by weight of p-toluenesulfonic acid·monohydrate and 292.5 parts by weight of toluene. While by-produced water was continuously removed out of the system under heating reflux, the resulting solution was reacted for 7.0 hours (internal temperature of 96 to 121 degree centigrade), and then cooled down to room temperature. The amount of water removed out of the system was 99.3% based on the theoretical amount of water to be generated. The reaction solution was washed with a base and subsequently washed with water, and then the reaction solution is removed toluene and a trace of water under heat and reduced pressure. Thereafter, 716.8 parts by weight of pentaerythritol 3-mercaptopropionic ester was obtained by filtering. Y.I. of the obtained pentaerythritol 3-mercaptopropionic ester was 1.0.

Viscosity of polymerizable composition before polymerization

87 parts by weight of m-xylylene diisocyanate, 0.01 weight part of dibutyltin dichloride as a curing catalyst, 0.18 parts by weight of ZELEC UN (product name, acid phosphate ester, a product of Stepan Co.) and 0. 10 weight part of Viosorb 583 (product name, ultraviolet absorber, a product of Kyodo Chemical Co., Ltd.) were mixed and dissolved at 20 degree centigrade. 113 parts by weight of the obtained pentaerythritol 3-mercaptopropionic ester was fed thereinto and mixed to give a uniform polymerizable composition before polymerization. The polymerizable composition before polymerization was kept at 20 degree centigrade and stirred for 7.0 hours. The viscosity at that time was 157 mPa·s.

Production of plastic lens

87 parts by weight of m-xylylene diisocyanate, 0.01 weight part of dibutyltin dichloride as a curing catalyst, 0.18 parts by weight of ZELEC UN (product name, acid phosphate ester, a product of Stepan Co.) and 0.10 weight part of Viosorb 583 (product name, ultraviolet absorber, a product of Kyodo Chemical Co., Ltd.) were mixed and dissolved at 20 degree centigrade. 113 parts by weight of the obtained pentaerythritol 3-mercaptopropionic ester was fed thereinto and mixed to give a uniform polymerizable composition before polymerization. The polymerizable composition before polymerization was degassed at 600 Pa for 1 hour, and then filtered using a 3-μm PTFE filter. Thereafter, the resulting solution was injected into a mold equipped with a glass mold and tapes. This mold was put into an oven and then gradually heated from 10 to 120 degree centigrade at which polymerization was conducted for 18 hours. After completion of polymerization, the mold was taken out from the oven and a resin was released from the mold. The obtained resin was additionally annealed at 130 degree centigrade for 4 hours. Y.I. of the obtained resin was 3.7 and the loss degree of transparency exhibiting transparency was 22. So, the resin was indicated with ○ regarding the loss degree of transparency.

Example 2

pentaerythritol 3-mercaptopropionic ester was synthesized in the same manner as in Example 1, except that a 3-mercaptopropionic acid with the purity of 96.1% containing 3.4% (in terms of area percentage) of 3-(3-mercaptopropanoylthio)propionic acid was used instead of the 3-mercaptopropionic acid used in Example 1. Y.I. of the obtained pentaerythritol 3-mercaptopropionic ester was 1.3. The viscosity of a polymerizable composition before polymerization with m-xylylene diisocyanate, which is containing the obtained pentaerythritol 3-mercaptopropionic ester and is obtained in the same manner as in Example 1, was 248 mPa·s. Furthermore, a plastic lens was prepared in the same manner as in Example 1. The evaluation results of the obtained plastic lens are shown in Table 1.

Example 3

pentaerythritol 3-mercaptopropionic ester was synthesized in the same manner as in Example 1, except that a 3-mercaptopropionic acid with the purity of 95.3% containing 4.2% (in terms of area percentage) of 3-(3-mercaptopropanoylthio)propionic acid was used instead of the 3-mercaptopropionic acid used in Example 1. Y.I. of the obtained pentaerythritol 3-mercaptopropionic ester was 1.8. The obtained pentaerythritol 3-mercaptopropionic ester was used and the viscosity of a polymerizable composition before polymerization with m-xylylene diisocyanate obtained in the same manner as in Example 1 was 288 mPa·s. Furthermore, a plastic lens was prepared in the same manner as in Example 1. The evaluation results of the obtained plastic lens are shown in Table 1.

Comparative Example 1

pentaerythritol 3-mercaptopropionic ester was synthesized in the same manner as in Example 1, except that a 3-mercaptopropionic acid with the purity of 90.2% containing 7.5% (in terms of area percentage) of 3-(3-mercaptopropanoylthio)propionic acid was used instead of the 3-mercaptopropionic acid used in Example 1. Y.I. of the obtained pentaerythritol 3-mercaptopropionic ester was 3.3. The obtained pentaerythritol 3-mercaptopropionic ester was used and the viscosity of a polymerizable composition before polymerization with m-xylylene diisocyanate obtained in the same manner as in Example 1 was 380 mPa·s. Furthermore, a plastic lens was prepared in the same manner as in Example 1. The evaluation results of the obtained plastic lens are shown in Table 1.

Comparative Example 2

pentaerythritol 3-mercaptopropionic ester was synthesized in the same manner as in Example 1, except that a 3-mercaptopropionic acid with the purity of 87.5% containing 11.1% (in terms of area percentage) of 3-(3-mercaptopropanoylthio)propionic acid was used instead of the 3-mercaptopropionic acid used in Example 1. Y.I. of the obtained pentaerythritol 3-mercaptopropionic ester was 5.2. The obtained pentaerythritol 3-mercaptopropionic ester was used and the viscosity of a polymerizable composition with m-xylylene diisocyanate obtained in the same manner as in Example 1 was 2,000 mPa·s or more. Furthermore, a plastic lens was attempted to be prepared in the same manner as in Example 1 but as a result, a plastic resin could not be obtained. This was because, since the viscosity of a polymerizable composition before polymerization was unusually high, filtering by using a 3-μm PTFE filter was extremely slow and it was difficult to inject the polymerizable composition into a mold equipped with a glass mold and tapes.

[Table 1]

| | Content of thioester (%) | Content of bispentaerythritol (wt %) | Viscosity of polymerizable composition (mPa·s) | Evaluation of thiol | Evaluation of lens | |
|---|---|---|---|---|---|---|
| | | | | Color Y.I. | Color Y.I. | Transparency Loss degree of transparency ($\leq 50$) |
| Example 1 | 0.2 | 4.7 | 157 | 1.0 | 3.7 | ○ (22) |
| Example 2 | 3.4 | 4.7 | 248 | 1.3 | 4.0 | ○ (39) |
| Example 3 | 4.2 | 4.7 | 288 | 1.8 | 4.0 | ○ (26) |
| Comparative Example 1 | 7.5 | 4.7 | 380 | 3.3 | 5.8 | × (65) |
| Comparative Example 2 | 11.1 | 4.7 | 2,000 or more | 5.2 | Unable to measure | Unable to measure |

INDUSTRIAL APPLICABILITY

According to the present invention, a polymerizable composition which is easily subjected to degassing, filtering of foreign substances and injection into a mold is obtained. Furthermore, using such a polymerizable composition, a high-quality urethane based plastic resin having excellent optical properties can be more economically produced.

**Claims**

1. A process for producing pentaerythritol mercaptocarboxylic ester, comprising:

    (i) purifying a mercaptocarboxylic acid to reduce the content of thioester formed by condensation of two molecules of the acid to not more than 5% in terms of area percentage, as determined by high-performance liquid chromatography with detection at 230 nm, the total area of the mercaptocarboxylic acid and thioester formed by intermolecular condensation of the acid being taken as 100%; and
    (ii) reacting pentaerythritol with the purified mercaptocarboxylic acid,

    wherein said mercaptocarboxylic acid is 3-mercaptopropionic acid.

2. The process for producing pentaerythritol mercaptocarboxylic ester as set forth in claim 1, in which the content of bispentaerythritol in said pentaerythritol is not more than 7 wt %, based on the total weight of pentaerythritol.

**Patentansprüche**

1. Verfahren zum Herstellen von Pentaerythritmercaptocarbonsäureester, umfassend:

    (i) Reinigen einer Mercaptocarbonsäure, um den Gehalt an Thioester, der durch die Kondensation von zwei Molekülen der Säure gebildet wird, auf nicht mehr als 5 % in Bezug auf einen Flächenprozentsatz, wie durch Hochleistungsflüssigchromatografie mit einer Detektion bei 230 nm bestimmt, zu verringern, wobei die Gesamtfläche der Mercaptocarbonsäure und des Thioesters, der durch intermolekulare Kondensation der Säure gebildet wird, als 100 % angenommen wird; und
    (ii) Umsetzen von Pentaerythrit mit der gereinigten Mercaptocarbonsäure,
    worin die Mercaptocarbonsäure 3-Mercaptopropionsäure ist.

2. Verfahren zum Herstellen von Pentaerythritmercaptocarbonsäureester nach Anspruch 1, bei dem der Gehalt an Bispentaerythrit in dem Pentaerythrit bezogen auf das Gesamtgewicht von Pentaerythrit nicht mehr als 7 Gew.-% ist.

**Revendications**

1. Procédé pour produire un ester mercaptocarboxylate de pentaérythritol, comprenant :

   (i) la purification d'un acide mercaptocarboxylique pour réduire la teneur en thioester formé par condensation de deux molécules de l'acide à au plus 5 % en termes de pourcentage de surface, comme déterminé par chromatographie liquide haute performance avec détection à 230 nm, la surface totale de l'acide mercaptocarboxylique et du thioester formé par condensation intermoléculaire de l'acide étant considérée être de 100 % ; et
   (ii) la réaction de pentaérythritol avec l'acide mercaptocarboxylique purifié,

   dans lequel ledit acide mercaptocarboxylique est l'acide 3-mercaptopropionique.

2. Procédé pour produire un ester mercaptocarboxylate de pentaérythritol selon la revendication 1, dans lequel la teneur en bispentaérythritol dudit pentaérythritol ne dépasse pas 7 % en poids, par rapport au poids total du pentaérythritol.

## REFERENCES CITED IN THE DESCRIPTION

**Patent documents cited in the description**

- JP S601985199016 A
- JP S601985217229 A
- JP S63198846213 A
- JP S3919649071 A
- JP S56198120530 A
- JP H101998120646 A
- JP 2005336104 A